# FASCICULE DE BREVET EUROPEEN

(11) **EP 4 494 171 B1**
(45) Date de publication et mention de la délivrance du brevet: **12.11.2025**
(21) Numéro de dépôt: 23712849.1
(22) Date de dépôt: 16.03.2023
(51) Int. Cl.: G21F 5/018, A61B 50/30, G21F 5/12, A61B 50/31, A61M 5/00

(54) **CONTENEUR BLINDÉ POUR LE TRANSPORT D'UN RÉCIPIENT CONTENANT UN PRODUIT RADIOACTIF**
ABSCHIRMBEHÄLTER ZUM TRANSPORT EINES RADIOAKTIVE PRODUKTE ENTHALTENDEN BEHÄLTERS
SHIELDING CONTAINER FOR TRANSPORTING A RECEPTACLE CONTAINING A RADIOACTIVE PRODUCT

(30) Priorité: 17.03.2022 FR 2202341
(43) Date de publication de la demande: 22.01.2025
(73) Titulaire: Lemer Pax, 44240 La Chapelle-sur-Erdre (FR)
(72) Inventeur: LEMER, Pierre-Marie, 44100 Nantes (FR)
(74) Mandataire: Jacobacci Coralis Harle
(86) Numéro de dépôt international: PCT/EP2023/056812
(87) Numéro de publication internationale: WO 2023/175108

(56) Documents cités:
- WO-A2-2017/207758
- US-A- 4 164 178
- US-A- 4 286 515
- US-A1- 2010 108 675

## Description

### Domaine technique de l'invention

La présente invention concerne le domaine technique des équipements assurant la protection des individus contre les rayonnements ionisants.

Elle concerne plus particulièrement un conteneur blindé réalisé en matériau radio-protecteur, conçu pour le transport d'un récipient contenant un produit radioactif, par exemple une seringue destinée à l'injection d'un produit radioactif à un patient.

### Etat de la technique

Dans le domaine ci-dessus on connait des conteneurs blindés adaptés au transport d'une seringue ou d'un flacon contenant un produit radiopharmaceutique qui est destiné à être injecté à un patient.

Ces conteneurs blindés, réalisés au moins partiellement en matériau radio-protecteur, peuvent se présenter sous la forme d'un réceptacle, formant le corps du conteneur, associé à un couvercle de conteneur.

Le réceptacle (ou corps de conteneur) comprend un élément de fond bordé par une paroi avant, une paroi arrière et deux parois d'extrémités, qui délimitent ensemble un volume de conditionnement pour la seringue et une ouverture de corps de conteneur.

Le couvercle de conteneur peut être solidaire du corps de conteneur par l'intermédiaire d'un axe d'articulation autorisant sa mobilité entre une position fermée, assurant la radioprotection recherchée, et une position ouverte, pour l'introduction ou l'extraction du récipient contenant le produit radiopharmaceutique.

Cependant de tels conteneurs blindés ont un poids relativement important (qui peut aller jusqu'à 15 Kg), et le couvercle de conteneur n'est pas facile à manœuvrer, avec les risques de pincement des doigts, voire de blessure, qui en découlent.

Les documents pertinents de l'art antérieur sont, par exemple, les suivants : US4164178A, US4286515A, et US2010/108675A1.

### Présentation de l'invention

Afin de remédier aux inconvénients précités de l'état de la technique, la présente invention propose un conteneur blindé, réalisé au moins partiellement en matériau radio-protecteur à l'encontre des rayonnements ionisants, pour le transport d'un récipient contenant un produit radioactif, par exemple une seringue destinée à l'injection d'un produit radioactif à un patient, lequel conteneur comprend :
- un corps de conteneur comprenant un élément de fond de corps de conteneur, bordé par une paroi avant de corps de conteneur, une paroi arrière de corps de conteneur et deux parois d'extrémités de corps de conteneur, délimitant ensemble un volume de conditionnement et une ouverture de corps de conteneur,
- un couvercle de conteneur comprenant un élément de dessus de couvercle bordé par un côté avant de couvercle, un côté arrière de couvercle et deux côtés d'extrémités de couvercle,
lequel couvercle de conteneur est solidaire dudit corps de conteneur par l'intermédiaire d'un axe d'articulation de couvercle autorisant sa mobilité par rapport audit corps de conteneur entre :
- une position fermée dans laquelle ledit couvercle de conteneur obture ladite ouverture de corps de conteneur, et
- une position ouverte dans laquelle ledit couvercle de conteneur libère ladite ouverture de corps de conteneur pour permettre l'accès audit volume de conditionnement,

ce conteneur blindé étant caractérisé par le fait qu'il comprend une structure de poignée (5) conçue pour permettre une prise en main pour le porter, laquelle structure de poignée (5) comprend un organe de préhension longitudinal, prolongé par deux bras latéraux de poignée solidaires des parois d'extrémité de corps de conteneur par l'intermédiaire d'un axe d'articulation de poignée,
lequel conteneur comprend encore des moyens de manœuvre pour manœuvrer ledit couvercle de conteneur entre lesdites positions ouverte et fermée,
lesquels moyens de manœuvre comprennent au moins une structure d'ergot solidaire d'un côté d'extrémité de couvercle, laquelle structure d'ergot est apte à se déplacer dans une lumière de guidage ménagée sur le bras latéral en regard de ladite structure de poignée,
laquelle lumière de guidage a une forme adaptée pour assurer la manœuvre en pivotement dudit couvercle de conteneur autour dudit axe d'articulation de couvercle, par l'intermédiaire de ladite structure d'ergot, cela par la manœuvre en pivotement de ladite structure de poignée autour dudit axe d'articulation de poignée.

La manœuvre du couvercle de conteneur s'effectue ainsi de manière contrôlée et sûre par une action de pivotement de la structure de poignée.

D'autres caractéristiques non limitatives et avantageuses du conteneur blindé conforme à l'invention, prises individuellement ou selon toutes les combinaisons techniquement possibles, sont les suivantes :
- la lumière de guidage est en forme d'arc de cercle dont le centre est situé du côté de l'axe d'articulation de couvercle ;
- l'une des extrémités de la lumière de guidage constitue un moyen de butée pour la structure d'ergot associée, de manière adaptée pour empêcher l'ouverture du couvercle de conteneur au-delà de 90° ;
- les deux bras latéraux de poignée comprennent une lumière de guidage coopérant chacune avec une structure d'ergot solidaire du côté d'extrémité de couvercle en correspondance du couvercle de conteneur ;
- la ou les structures d'ergot sont ménagées sur le côté d'extrémité de couvercle associé, entre l'axe d'articulation de couvercle et le côté avant de couvercle ;
- l'axe d'articulation de poignée est ménagé à proximité de la paroi avant de corps de conteneur ;
- le corps de conteneur comprend un plan longitudinal médian qui s'étend parallèlement audites parois avant et arrière de corps de conteneur et qui traverse lesdites parois d'extrémités de corps de conteneur dans leur zone centrale,
   et ledit organe de préhension longitudinal de la structure de poignée s'étend, en position fermée du couvercle de conteneur, dans ledit plan longitudinal médian ou approximativement dans ledit plan longitudinal médian ;
- l'élément de fond de corps de conteneur, la paroi avant de corps de conteneur, la paroi arrière de corps de conteneur, les parois d'extrémités de corps de conteneur, et l'élément de dessus du couvercle, comprennent une âme en plomb recouverte par des parements intérieur et extérieur en matériau moins radio-protecteur que le plomb, tel que l'acier inoxydable par exemple ;
- le conteneur blindé comprend un plateau rapporté contre la face externe de l'élément de fond de corps de conteneur, lequel plateau comprend une extension anti-basculement qui s'étend en saillie du plan de la paroi arrière de corps de conteneur, agencée pour éviter le basculement dudit conteneur blindé en fin de course d'ouverture du couvercle de conteneur ;
- le conteneur blindé comprend un support de conditionnement amovible et étanche aux liquides, disposé dans le volume de conditionnement, en appui sur l'élément de fond de corps de conteneur, lequel support de conditionnement est adapté pour la réception et le calage d'un récipient contenant un produit radioactif.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée en référence aux dessins qui illustrent des formes, non limitatives, de réalisation de l'invention et où :
[Fig. 1] est une vue en perspective d'une forme de réalisation possible d'un conteneur blindé conforme à l'invention, représenté ici avec son couvercle de conteneur en position fermée ;
[Fig. 2] est une vue en perspective du conteneur blindé illustré sur la figure 1, représenté ici avec son couvercle de conteneur en position ouverte ;
[Fig. 3] montre le conteneur blindé illustré sur la figure 2, vu en perspective selon un autre angle ;
[Fig. 4] est une vue de côté du conteneur blindé illustré sur les figures 1 à 3, représenté ici avec son couvercle de conteneur en position fermée ;
[Fig. 5] est une vue de côté du conteneur blindé illustré sur les figures 1 à 4, représenté ici avec son couvercle de conteneur en position ouverte ;
[Fig. 6] est une vue de face du conteneur blindé illustré sur les figures 1 à 5, représenté ici avec son couvercle de conteneur en position ouverte ;
[Fig. 7] est une vue en coupe selon le plan de coupe 7-7 du conteneur blindé illustré sur la figure 6.

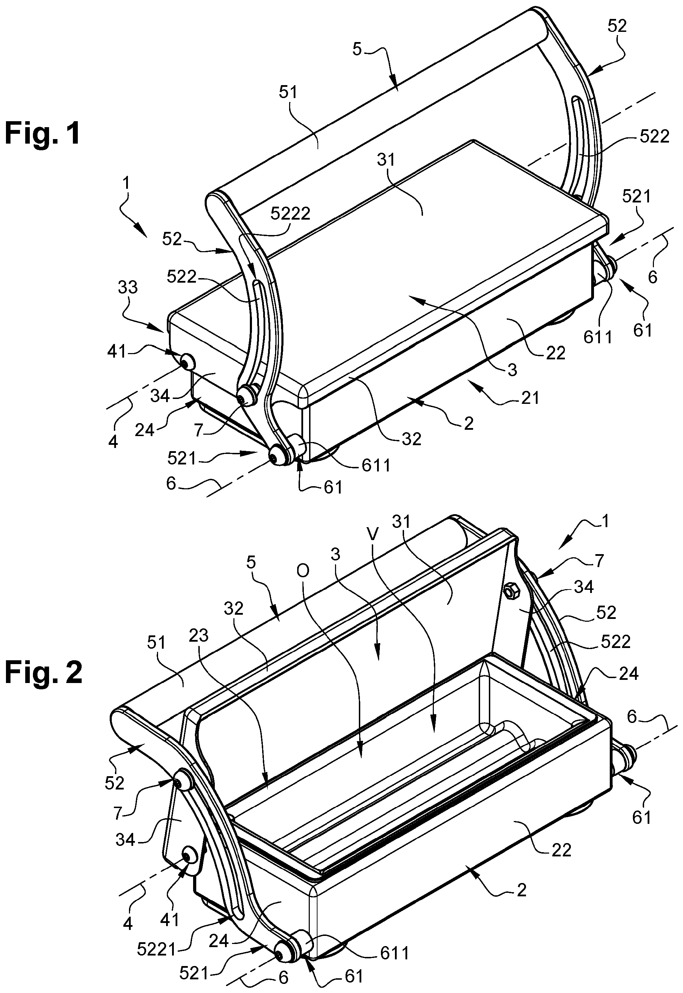

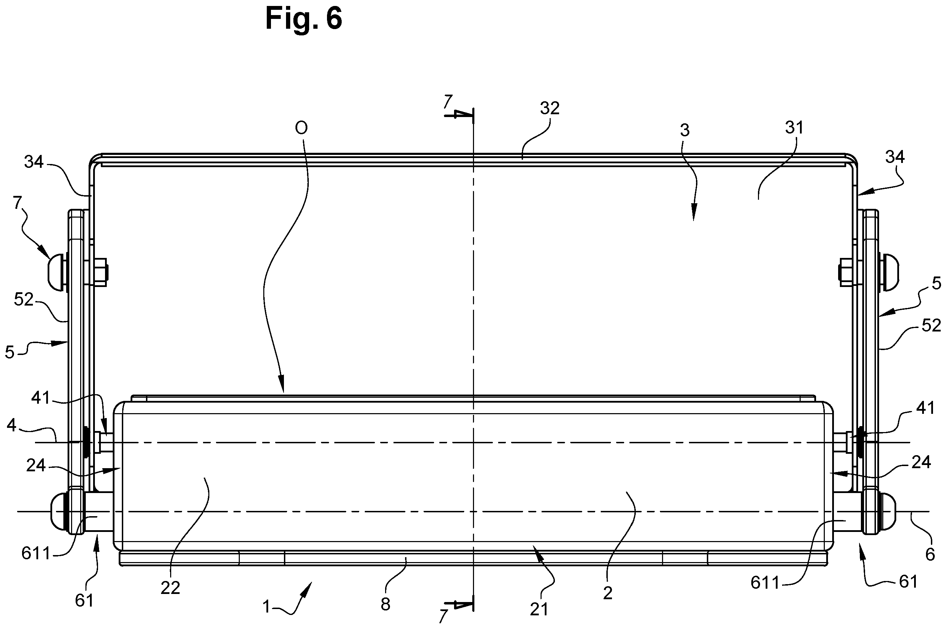

Le conteneur blindé 1 illustré sur les figures 1 à 7 se présente sous la forme générale d'une valisette adaptée pour le transport d'un récipient (non représenté) contenant un produit radioactif, par exemple une seringue ou un flacon contenant un produit radiopharmaceutique destiné à être injecté à un patient.

Ce conteneur blindé 1 comprend:
- un corps de conteneur 2,
- un couvercle de conteneur 3, articulé sur le corps de conteneur 2 autour d'un axe d'articulation de couvercle 4, et
- une structure de poignée 5 qui permet une prise en main pour porter le conteneur blindé 1, et qui permet également une manœuvre du couvercle de conteneur 3 entre ses positions ouverte et fermée par rapport au corps de conteneur 2, comme expliqué plus loin dans la description.

Le corps de conteneur 2 comprend un élément de fond de corps de conteneur 21, plan, bordé par une paroi avant de corps de conteneur 22, une paroi arrière de corps de conteneur 23 et deux parois d'extrémités de corps de conteneur 24.

La paroi avant de corps de conteneur 22, la paroi arrière de corps de conteneur 23 et les deux parois d'extrémités de corps de conteneur 24 ont toutes la même hauteur. Elles s'étendent du même côté par rapport au plan de l'élément de fond de corps de conteneur 21 et elles forment ensemble une ceinture de parois périphériques.

L'élément de fond de corps de conteneur 21 a une forme générale rectangulaire ; la paroi avant de corps de conteneur 22 et la paroi arrière de corps de conteneur 23 sont parallèles entre elles ; et de leur côté, les deux parois d'extrémités de corps de conteneur 24 sont également parallèles entre elles.
Le corps de conteneur 2 comprend un plan longitudinal médian P qui s'étend parallèlement aux parois avant et arrière de corps de conteneur 22, 23 et qui traverse les parois d'extrémités de corps de conteneur 24 dans leur zone centrale.

Les différentes parties constitutives 21, 22, 22 et 24 du corps de conteneur 2 délimitent ensemble un volume de conditionnement V et une ouverture de corps de conteneur O.

De son côté, le couvercle de conteneur 3 comprend un élément de dessus de couvercle 31, plan, bordé par un côté avant de couvercle 32, un côté arrière de couvercle 33 et deux côtés d'extrémités de couvercle 34.

Le côté avant de couvercle 32, le côté arrière de couvercle 33 et les deux côtés d'extrémités de couvercle 34 forment ensemble un rebord périphérique qui s'étend d'un côté du plan de l'élément de dessus de couvercle 31.

L'élément de dessus de couvercle 31 a une forme générale rectangulaire ; le côté avant de couvercle 32 et le côté arrière de couvercle 33 sont parallèles entre eux ; et de leur côté, les deux côtés d'extrémités de couvercle 34 sont également parallèles entre eux.

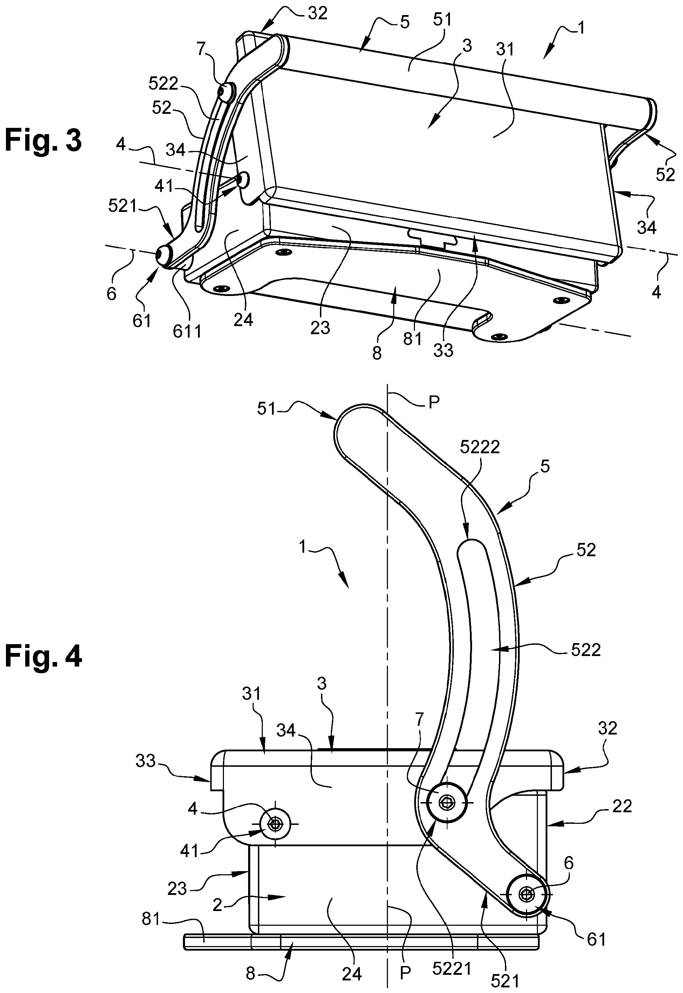

Le couvercle de conteneur 3 est solidaire du corps de conteneur 2 par l'intermédiaire de l'axe d'articulation de couvercle 4 qui autorise sa mobilité entre :
- une position fermée (visible sur les figures 1 et 4) dans laquelle le couvercle de conteneur 3 obture l'ouverture de corps de conteneur O, et
- une position ouverte (visible sur les figures 2, 3, 5, 6 et 7) dans laquelle le couvercle de conteneur 3 libère l'ouverture de corps de conteneur O pour permettre l'accès au volume de conditionnement V.

En position fermée, l'élément de dessus de couvercle 31 du couvercle de conteneur 3 vient reposer sur la bordure supérieure de la ceinture de parois périphériques 22, 23, 24 du corps de conteneur 2 ; et le rebord périphérique formé par le côté avant de couvercle 32, le côté arrière de couvercle 33 et les deux côtés d'extrémités de couvercle 34 vient entourer cette ceinture de parois périphériques 22, 23, 24.

L'axe d'articulation de couvercle 4 est ménagé à proximité de la paroi arrière de corps de conteneur 23 et du côté arrière de couvercle 33. Il est matérialisé par deux structures pivot de couvercle 41 reliant, de part et d'autre du conteneur 1, un côté d'extrémité de couvercle 34 et une paroi d'extrémité de corps de conteneur 24.

Chaque structure pivot de couvercle 41 peut consister en un système de liaison de type vis-écrou qui traverse le côté d'extrémité de couvercle 34 et la paroi d'extrémité de corps de conteneur 24.

La structure de poignée 5 du conteneur 1 comprend un organe de préhension longitudinal 51, prolongé par deux bras latéraux de poignée 52 solidaires des parois d'extrémité de corps de conteneur 24.

Comme précisé ci-dessus, la structure de poignée 5 est adaptée non seulement pour permettre la préhension du conteneur 1, mais aussi pour assurer la manœuvre du couvercle de conteneur 3 entre ses positions ouverte et fermée.

Pour cela :
- les extrémités 521 des deux bras latéraux de poignée 52 sont solidaires des parois d'extrémité de corps de conteneur 24 par l'intermédiaire d'un axe d'articulation de poignée 6, et
- les moyens de manœuvre du couvercle de conteneur 3 comprennent, de chaque côté du conteneur 1, une structure d'ergot 7 solidaire d'un côté d'extrémité de couvercle 34, qui est apte à se déplacer dans une lumière de guidage 522 ménagée sur le bras latéral 52 en regard de la structure de poignée 5.

Les couples structure d'ergot 7 / lumière de guidage 522 sont identiques de chaque côté du conteneur 1.

Le positionnement des structures d'ergot 7 sur les côtés d'extrémités de couvercle 34, et la forme des lumières de guidage 522, sont adaptés pour assurer la manœuvre en pivotement du couvercle de conteneur 3 autour de l'axe d'articulation de couvercle 4, par l'intermédiaire des structures d'ergots 7, cela par la manœuvre en pivotement de la structure de poignée 5 autour de l'axe d'articulation de poignée 6.

Dans ce cadre, les structures d'ergot 7 sont ménagées sur le côté d'extrémité de couvercle 34 associé, entre l'axe d'articulation de couvercle 4 et le côté avant de couvercle 32.

D'autre part, chaque lumière de guidage 522 est en forme d'arc de cercle dont le centre est situé du côté de l'axe d'articulation de couvercle 4.

L'axe d'articulation de poignée 6 est matérialisé par deux structures pivot de poignée 61 reliant, de chaque côté du conteneur 1, l'extrémité 521 du bras latéral de poignée 52 et la paroi d'extrémité de corps de conteneur 24 en regard.

Chaque structure pivot de poignée 61 peut consister en un système de liaison de type vis-écrou qui traverse le bras latéral de poignée 52 (au niveau de son extrémité 521) et la paroi d'extrémité de corps de conteneur 24 en regard. Une rondelle entretoise 611 est prévue entre l'extrémité 521 des bras latéraux de poignée 52 et la paroi d'extrémité de corps de conteneur 24 en regard, pour tenir compte de la différence de longueur entre le couvercle de conteneur 3 et le corps de conteneur 2.

Comme on peut le voir sur les figures annexées, l'axe d'articulation de poignée 6 est ménagé à proximité de la paroi avant de corps de conteneur 22.

Chaque structure d'ergot 7 peut consister en un système de type vis-écrou qui traverse le côté d'extrémité de couvercle 34 en regard. L'écrou de ce système vis-écrou est ici positionné à l'intérieur du couvercle de conteneur 3 ; le corps de la vis constitue l'élément qui circule dans la lumière de guidage 522 associée ; et la tête de vis constitue un organe de butée évitant la désolidarisation entre le bras latéral de poignée 52 et le couvercle de conteneur 3.

Pour des raisons de stabilité et de fluidité de mouvement, notamment, les deux côtés du conteneur 1 sont de préférence équipés d'un couple structure d'ergot 7 / lumière de guidage 522, tel qu'illustré sur les figures ; cependant, le cas échéant, dans une variante de réalisation, seul l'un des deux côtés du conteneur 1 peut être équipé d'un tel couple structure d'ergot 7 / lumière de guidage 522.

Les deux extrémités des lumières de guidage 522 servent de moyen de butée pour les structures d'ergot 7 et définissent les positions stables ouverte et fermée du couvercle de conteneur 3
Plus précisément, l'extrémité 5221 de la lumière de guidage 522, qui est située du côté de l'axe d'articulation de poignée 6, sert de moyen de butée pour les structures d'ergot 7 afin de définir la position stable fermée du couvercle de conteneur 3 (figure 1 et 4).

Pour faciliter et optimiser les opérations de transport à la main du conteneur 1, dans cette position stable fermée, l'organe de préhension longitudinal 51 de la structure de poignée 5 s'étend dans le plan longitudinal médian P (ou approximativement dans ce plan longitudinal médian P) du corps de conteneur 2.

De son côté, l'extrémité 5222 de la lumière de guidage 522, qui est située à distance de l'axe d'articulation de poignée 6, sert de moyen de butée pour les structures d'ergot 7 afin de définir la position stable ouverte du couvercle de conteneur 3 (figures 2, 3, 5, 6 et 7).

On remarque que l'extrémité 5222 de la lumière de guidage 522 empêche l'ouverture du couvercle de conteneur 3 au-delà de 90° de manière à assurer la stabilité du conteneur 3 et limiter ses risques de basculement lorsqu'il est posé sur un support.
Plus précisément, cette extrémité 5222 de la lumière de guidage 522 limite l'ouverture du couvercle de conteneur 3 à un secteur angulaire légèrement inférieur à 90° (de l'ordre de 85 à 88°).

Toujours dans un but d'optimisation de stabilité, le conteneur blindé 1 comprend un plateau 8 rapporté contre la face externe de l'élément de fond de corps de conteneur 21 ; et ce plateau 8 comprend une extension anti-basculement 81 qui s'étend en saillie par rapport au plan de la paroi arrière de corps de conteneur 23.

Ce plateau 8 est fixé par tout moyen approprié contre la face externe (ou de dessous) de l'élément de fond de corps de conteneur 21, par exemple par vissage.

On comprend que l'extension anti-basculement 81, en saillie du côté où le couvercle de conteneur 3 est articulé sur le corps de conteneur 2, évite ou tout au moins limite le risque de basculement du conteneur blindé 1 en fin de course d'ouverture du couvercle de conteneur 3.

Le conteneur 1 est réalisé au moins partiellement en matériau radio-protecteur, de manière adaptée, lorsqu'il est en position fermée, pour protéger l'environnement, et en particulier les individus à proximité, à l'encontre des rayonnements ionisants émis par le produit radioactif conditionné.

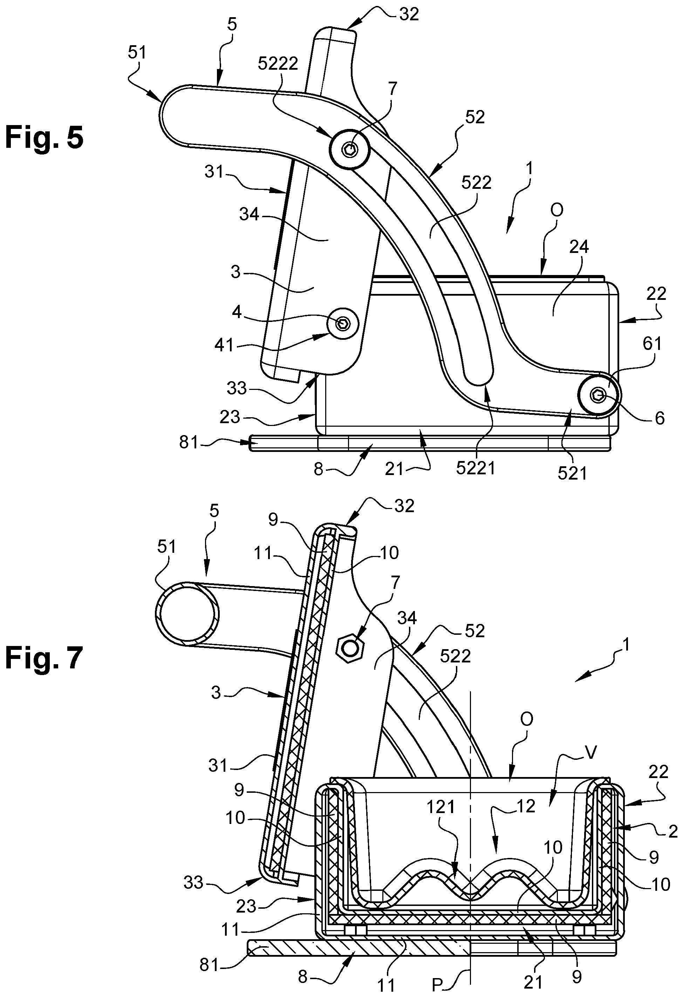

Tel qu'illustré sur la vue en coupe de la figure 7, l'élément de fond de corps de conteneur 21, la paroi avant de corps de conteneur 22, la paroi arrière de corps de conteneur 23, les parois d'extrémités de corps de conteneur 24, et l'élément de dessus de couvercle 31, peuvent comprendre une âme en plomb 9 recouverte par des parements intérieurs 10 et des parement extérieurs 11 en matériau mécaniquement résistant et moins radio-protecteur que le plomb (par exemple en forme de plaques de recouvrement en acier inoxydable).

Ici, le rebord périphérique du couvercle de conteneur 3 formé par le côté avant de couvercle 32, le côté arrière de couvercle 33 et les deux côtés d'extrémités de couvercle 34 sont réalisés uniquement en acier inoxydable.

Dans une variante de réalisation le conteneur blindé 1 peut être réalisé en tungstène massif.

Toujours sur la vue en coupe de la figure 7, on remarque que le conteneur blindé 1 peut comprendre un support de conditionnement 12 disposé dans le volume de conditionnement V, adapté pour la réception et le calage d'un récipient (par exemple en forme de seringue ou de flacon) contenant le produit radioactif.

Ce support de conditionnement 12 est disposé en appui sur l'élément de fond de corps de conteneur 21 ; et il est prévu amovible et étanche aux liquides.

Le support de conditionnement 12 peut se présenter sous la forme d'un bac de rétention adapté à recueillir les éventuelles fuites de produit radioactif et dont la partie de fond 121 est adaptée pour la réception et le calage du récipient contenant le produit radioactif.
Dans le mode de réalisation illustré, la partie de fond 121 du support de conditionnement 12 est en forme de berceau adapté pour la réception d'un récipient de type seringue.

## Revendications

1. Conteneur (1) blindé, réalisé au moins partiellement en matériau radio-protecteur à l'encontre des rayonnements ionisants, conçu pour le transport d'un récipient contenant un produit radioactif, par exemple une seringue destinée à l'injection d'un produit radioactif à un patient, lequel conteneur (1) comprend :
- un corps de conteneur (2) comprenant un élément de fond de corps de conteneur (21), bordé par une paroi avant de corps de conteneur (22), une paroi arrière de corps de conteneur (23) et deux parois d'extrémités de corps de conteneur (24), délimitant ensemble un volume de conditionnement (V) et une ouverture de corps de conteneur (O),
- un couvercle de conteneur (3) comprenant un élément de dessus de couvercle (31) bordé par un côté avant de couvercle (32), un côté arrière de couvercle (33) et deux côté d'extrémités de couvercle (34),
lequel couvercle de conteneur (3) est solidaire dudit corps de conteneur (2) par l'intermédiaire d'un axe d'articulation de couvercle (4) autorisant sa mobilité par rapport audit corps de conteneur (2) entre :
- une position fermée dans laquelle ledit couvercle de conteneur (3) obture ladite ouverture de corps de conteneur (O), et
- une position ouverte dans laquelle ledit couvercle de conteneur (3) libère ladite ouverture de corps de conteneur (O) pour permettre l'accès audit volume de conditionnement (V),
**caractérisé en ce que** ledit conteneur (1) blindé comprend une structure de poignée (5) conçue pour permettre une prise en main pour le porter, laquelle structure de poignée (5) comprend un organe de préhension longitudinal (51), prolongé par deux bras latéraux de poignée (52) solidaires des parois d'extrémité de corps de conteneur (24) par l'intermédiaire d'un axe d'articulation de poignée (6),
lequel conteneur (1) comprend encore des moyens de manœuvre (7, 522) pour manœuvrer ledit couvercle de conteneur (3) entre lesdites positions ouverte et fermée,
lesquels moyens de manœuvre comprennent au moins une structure d'ergot (7) solidaire d'un côté d'extrémité de couvercle (34), laquelle structure d'ergot (7) est apte à se déplacer dans une lumière de guidage (522) ménagée sur le bras latéral (52) en regard de ladite structure de poignée (5),
laquelle lumière de guidage (522) a une forme adaptée pour assurer la manœuvre en pivotement dudit couvercle de conteneur (3) autour dudit axe d'articulation de couvercle (4), par l'intermédiaire de ladite structure d'ergot (7), cela par la manœuvre en pivotement de ladite structure de poignée (5) autour dudit axe d'articulation de poignée (6).

2. Conteneur (1) blindé selon la revendication 1, **caractérisé en ce que** ladite lumière de guidage (522) est en forme d'arc de cercle dont le centre est situé du côté dudit axe d'articulation de couvercle (4).

3. Conteneur (1) blindé selon l'une quelconque des revendications 1 ou 2, **caractérisé en ce que** l'une des extrémités (5222) de ladite lumière de guidage (522) constitue un moyen de butée pour la structure d'ergot (7) associée, de manière adaptée pour empêcher l'ouverture dudit couvercle de conteneur (3) au-delà de 90°.

4. Conteneur (1) blindé selon l'une quelconque des revendications 1 à 3, **caractérisé en ce que** les deux bras latéraux de poignée (52) comprennent une lumière de guidage (522) coopérant chacune avec une structure d'ergot (7) solidaire du côté d'extrémité de couvercle (34) en correspondance dudit couvercle de conteneur (3).

5. Conteneur (1) blindé selon l'une quelconque des revendications 1 à 4, **caractérisé en ce que** ladite ou lesdites structures d'ergot (7) sont ménagées sur le côté d'extrémité de couvercle (34) associé, entre ledit axe d'articulation de couvercle (4) et ledit côté avant de couvercle (32).

6. Conteneur (1) blindé selon l'une quelconque des revendications 1 à 5, **caractérisé en ce que** ledit axe d'articulation de poignée (6) est ménagé à proximité de ladite paroi avant de corps de conteneur (22).

7. Conteneur (1) blindé selon l'une quelconque des revendications 1 à 6, **caractérisé en ce que** ledit corps de conteneur (2) comprend un plan longitudinal médian (P) qui s'étend parallèlement audites parois avant et arrière de corps de conteneur (22, 23) et qui traverse lesdites parois d'extrémités de corps de conteneur (24) dans leur zone centrale,
et **en ce que** ledit organe de préhension longitudinal (51) de ladite structure de poignée (5) s'étend, en position fermée du couvercle de conteneur (3), dans ledit plan longitudinal médian (P) ou approximativement dans ledit plan longitudinal médian (P).

8. Conteneur (1) blindé selon l'une quelconque des revendications 1 à 7, **caractérisé en ce que** ledit élément de fond de corps de conteneur (21), ladite paroi avant de corps de conteneur (22), ladite paroi arrière de corps de conteneur (23), lesdites parois d'extrémités de corps de conteneur (24) et ledit élément de dessus de couvercle (31), comprennent une âme en plomb (9) recouverte par des parements intérieur (10) et extérieur (11) en matériau moins radio-protecteur que le plomb, tel que l'acier inoxydable par exemple.

9. Conteneur (1) blindé selon l'une quelconque des revendications 1 à 8, **caractérisé en ce qu'**il comprend un plateau (8) rapporté contre la face externe de l'élément de fond du corps de conteneur (21), lequel plateau (8) comprend une extension anti-basculement (81) qui s'étend en saillie du plan de la paroi arrière de corps de conteneur (23), agencée pour éviter le basculement dudit conteneur blindé (1) en fin de course d'ouverture du couvercle de conteneur (3).

10. Conteneur (1) blindé selon l'une quelconque des revendications 1 à 9, **caractérisé en ce qu'**il comprend un support de conditionnement (12) amovible et étanche aux liquides, disposé dans ledit volume de conditionnement (V), en appui sur ledit élément de fond de corps de conteneur (21), lequel support de conditionnement (12) est adapté pour la réception et le calage d'un récipient contenant un produit radioactif.

## Patentansprüche

1. Abschirmbehälter (1), der mindestens teilweise aus einem vor den ionisierenden Strahlen schützenden Strahlenschutzmaterial gefertigt ist, für den Transport eines Behälters ausgelegt ist, der ein radioaktives Produkt enthält, wie zum Beispiel eine Spritze, die dazu bestimmt ist, einem Patienten ein radioaktives Produkt einzuspritzen,
wobei der Abschirmbehälter (1)
- einen Behälterkörper (2) mit einem Behälterkörperbodenelement (21), das von einer Behälterkörpervorderwand (22), einer Behälterkörperrückwand (23) und zwei Behälterkörperendwänden (24) eingefaßt ist, die zusammen ein Verpackungsvolumen (V) und eine Behälterkörperöffnung (O) definieren,
- einen Behälterdeckel (3) mit einem Deckeloberelement (31), das von einer vorderen Deckelseite (32), einer hinteren Deckelseite (33) und zwei Deckelendseiten (34) eingefaßt ist,
aufweist,
wobei der Behälterdeckel (3) mit dem Behälterkörper (2) durch eine Deckelgelenkachse (4) verbunden ist, die dessen Beweglichkeit gegenüber dem Behälterkörper (2) zwischen
- einer Verschlußposition, in der der Behälterdeckel (3) die Behälterkörperöffnung (O) verschließt, und
- einer Öffnungsposition, in der der Behälterdeckel (3) die Behälterkörperöffnung (O) freigibt, um Zugang zum Verpackungsvolumen (V) zu gewähren,
ermöglicht,
**dadurch gekennzeichnet, daß** der Abschirmbehälter (1) eine Griffstruktur (5) aufweist, die dazu ausgelegt ist, einem Träger ein In-die-Hand-Nehmen zu ermöglichen, wobei die Griffstruktur (5) ein Längsgrifforgan (51) aufweist, das durch zwei seitliche Griffarme (52) verlängert ist, die mit den Behälterkörperendwänden (24) durch eine Griffgelenkachse (6) verbunden sind,
wobei der Behälter (1) außerdem Betätigungsmittel (7, 522) zum Bewegen des Behälterdeckels (3) zwischen der Öffnungs- und der Verschlußposition aufweist,
wobei die Betätigungsmittel mindestens eine mit einer Deckelendseite (34) verbundene Nockenstruktur (7) aufweisen und wobei sich die Nockenstruktur (7) in einer im seitlichen Arm (52) gegenüber der Griffstruktur (5) ausgebildeten Führungsöffnung (522) bewegen kann,
wobei die Führungsöffnung (522) eine so angepaßte Form hat, daß die Schwenkbewegung des Behälterdeckels (3) um die Deckelgelenkachse (4) mittels der Nockenstruktur (7) und diese wiederum durch die Schwenkbewegung der Griffstruktur (5) um die Griffgelenkachse (6) sichergestellt ist.

2. Abschirmbehälter (1) gemäß Anspruch 1, **dadurch gekennzeichnet, daß** die Führungsöffnung (522) die Form eines Kreisbogens hat, dessen Mittelpunkt auf der Seite der Deckelgelenkachse (4) gelegen ist.

3. Abschirmbehälter (1) gemäß Anspruch 1 oder 2, **dadurch gekennzeichnet, daß** eins der Enden (5222) der Führungsöffnung (522) in angepaßter Weise ein Anschlagsmittel für die zugehörige Nockenstruktur (7) bildet, um ein Öffnen des Behälterdeckels (3) über 90° hinaus zu verhindern.

4. Abschirmbehälter (1) gemäß einem der Ansprüche 1 bis 3, **dadurch gekennzeichnet, daß** die beiden seitlichen Griffarme (52) eine Führungsöffnung (522) aufweisen, die jeweils mit einer mit der entsprechenden Deckelendseite (34) des Behälterdeckels (3) verbundenen Nockenstruktur (7) zusammenwirken.

5. Abschirmbehälter (1) gemäß einem der Ansprüche 1 bis 4, **dadurch gekennzeichnet, daß** die Nockenstruktur beziehungsweise Nockenstrukturen (7) auf der zugehörigen Deckelendseite (34) zwischen der Deckelgelenkachse (4) und der vorderen Deckelseite (32) eingerichtet ist beziehungsweise sind.

6. Abschirmbehälter (1) gemäß einem der Ansprüche 1 bis 5, **dadurch gekennzeichnet, daß** die Griffgelenkachse (6) in der Nähe der Behälterkörpervorderwand (22) angebracht ist.

7. Abschirmbehälter (1) gemäß einem der Ansprüche 1 bis 6, **dadurch gekennzeichnet, daß** der Behälterkörper (2) eine Längsmittelebene (P) aufweist, die sich parallel zur Vorder- und zur Rückwand (22, 23) des Behälterkörpers erstreckt und die die Behälterkörperendwände (24) in deren Mittelbereich durchquert,
und daß sich das Längsgrifforgan (51) der Griffstruktur (5) in der Verschlußposition des Behälterdeckels (3) in der Längsmittelebene (P) oder näherungsweise in der Längsmittelebene (P) erstreckt.

8. Abschirmbehälter (1) gemäß einem der Ansprüche 1 bis 7, **dadurch gekennzeichnet, daß** das Behälterkörperbodenelement (21), die Behälterkörpervorderwand (22), die Behälterkörperrückwand (23), die beiden Behälterkörperendwände (24) und das Deckeloberelement (31) eine mit einer inneren (10) und einer äußeren (11) Abdeckung aus einem weniger als Blei strahlungssicheren Material wie etwa rostfreier Stahl versehene Bleiseele (9) aufweisen.

9. Abschirmbehälter (1) gemäß einem der Ansprüche 1 bis 8, **dadurch gekennzeichnet, daß** er eine an der Außenseite des Behälterkörperbodenelements (21) angebrachte Platte (8) aufweist, wobei die Platte (8) eine Kippschutzerweiterung (81) aufweist, die aus der Ebene der Behälterkörperrückwand (23) vorsteht und dazu ausgelegt ist, ein Kippen des Abschirmbehälters (1) am Ende der Öffnungsstrecke des Behälterdeckels (3) zu verhindern.

10. Abschirmbehälter (1) gemäß einem der Ansprüche 1 bis 9, **dadurch gekennzeichnet, daß** er einen herausnehmbaren und flüssigkeitsdichten Verpackungsträger (12) aufweist, der im Verpackungsvolumen (V) in Anlage an das Behälterkörperbodenelement (21) angeordnet ist, wobei der Verpackungsträger (12) für das Aufnehmen und Festkeilen eines ein radioaktives Produkt enthaltenden Behälters ausgelegt ist.

## Claims

1. A shielded container (1), made at least partially of a radioprotective material for protection against ionising radiations, for transporting a vessel containing a radioactive product, for example a syringe for injecting a radioactive product into a patient, said container (1) comprising:
- a container body (2) comprising a container body bottom element (21), bordered by a container body front wall (22), a container body rear wall (23) and two container body end walls (24), delimiting together a packaging volume (V) and a container body opening (O),
- a container lid (3) comprising a lid top element (31) bordered by a lid front side (32), a lid rear side (33) and two lid end sides (34),
said container lid (3) being rigidly attached to said container body (2) via a lid hinge axis (4) allowing it to move with respect to said container body (2) between:
- a closed position in which said container body (3) closes said container body opening (O), and
- an open position in which said container lid (3) clears said container body opening (O) to allow access to said packaging volume (V),
**characterized in that** said shielded container (1) comprises a handle structure (5) designed to be gripped for carrying it, said handle structure (5) comprising a longitudinal grip member (51), extended by two side handle arms (52) rigidly attached to the container body end walls (24) via a handle hinge axis (6),
said container (1) also comprising manoeuvring means (7, 522) for manoeuvring said container lid (3) between said open and closed positions,
said manoeuvring means comprising at least one lug structure (7) rigidly attached to one lid end side (34), said lug structure (7) being able to move in a guiding slot (522) formed in the side arm (52) opposite said handle structure (5),
said guiding slot (522) being so shaped to pivotally manoeuvre said container lid (3) about said lid hinge axis (4), through said lug structure (7), by pivotally manoeuvring said handle structure (5) about said handle hinge axis (6).

2. The shielded container (1) according to claim 1, **characterized in that** said guiding slot (522) has the shape of an arc of a circle whose centre is located on the side of said lid hinge axis (4).

3. The shielded container (1) according to any one of claims 1 or 2, **characterized in that** one of the ends (5222) of said guiding slot (522) forms a stop means for the associated lug structure (7), in a suitable way to prevent said container lid (3) from opening beyond 90°.

4. The shielded container (1) according to any one of claims 1 to 3, **characterized in that** the two side handle arms (52) comprise a guiding slot (522) each cooperating with a lug structure (7) rigidly attached to the corresponding lid end side (34) of said container lid (3).

5. The shielded container (1) according to any one of claims 1 to 4, **characterized in that** said lug structure(s) (7) are arranged on the associated lid end side (34), between said lid hinge axis (4) and said lid front side (32).

6. The shielded container (1) according to any one of claims 1 to 5, **characterized in that** said handle hinge axis (6) is arranged near said container body front wall (22).

7. The shielded container (1) according to any one of claims 1 to 6, **characterized in that** said container body (2) comprises a longitudinal median plane (P) that extends parallel to said container body front and rear walls (22, 23) and that passes through the container body end walls (24) in their central area,
and **in that** said longitudinal grip member (51) of said handle structure (5) extends, in closed position of the container lid (3), in said longitudinal median plane (P) or approximately in said longitudinal median plane (P).

8. The shielded container (1) according to any one of claims 1 to 7, **characterized in that** said container body bottom element (21), said container body front wall (22), said container body rear wall (23), said container body end walls (24) and said lid top element (31) comprise a lead core (9) covered by inner (10) and outer (11) linings made of a material that is less radioprotective than lead, such as stainless steel for example.

9. The shielded container (1) according to any one of claims 1 to 8, **characterized in that** it comprises a plate (8) added against the outer face of the container body bottom element (21), said plate (8) comprising an anti-tilt extension (81) that projects from the plane of the container body rear wall (23), arranged to prevent said shielded container (1) from tipping over at the end of the container lid (3) opening stroke.

10. The shielded container (1) according to any one of claims 1 to 9, **characterized in that** it comprises a removable, liquid-tight packaging support (12) arranged in said packaging volume (V), in rest on the container body bottom element (21), said packaging support (12) being adapted to receive and wedge a vessel containing a radioactive product.
